Europäisches Patentamt

European Patent Office

Office européen des brevets

(11) Numéro de publication: **0 292 341**
**A1**

(12) **DEMANDE DE BREVET EUROPEEN**

(21) Numéro de dépôt: **88400872.3**

(22) Date de dépôt: **12.04.88**

(51) Int. Cl.⁴: **C 08 F 20/06**
**A 61 L 15/00**

(30) Priorité: **14.04.87 FR 8705250**

(43) Date de publication de la demande:
**23.11.88  Bulletin  88/47**

(84) Etats contractants désignés:
**BE CH DE FR GB LI**

(71) Demandeur: **SOCIETE FRANCAISE HOECHST Société anonyme dite:**
**3, avenue du Général de Gaulle**
**F-92800 Puteaux  (FR)**

(72) Inventeur: **Cabestany, Jean**
**127 Boulevard Maxime Gorky**
**F-93240 Stains  (FR)**

(74) Mandataire: **Santarelli, Marc**
**Cabinet Rinuy et Santarelli 14, avenue de la Grande Armée**
**F-75017 Paris  (FR)**

(54) **Polymère hydrophile à base d'acide acrylique et d'acrylate de métal alcalin, son procédé de préparation et son application comme agent absorbant, en particulier d'une solution physiologique.**

㊼ Ce polymère est à base d'acide acrylique et d'acrylate de métal alcalin ; il présente une capacité d'absorption de solution physiologique de 50 à 70 g/g ne renfermant que 20 à 45 % molaires d'acide acrylique et 80 à 55 % d'acrylate de potassium.

Son procédé de préparation est un procédé de polymérisation en suspension eau dans huile par introduction lente sous agitation dans la phase huile renfermant un colloïde protecteur d'une solution obtenue à partir d'une solution aqueuse d'un ou plusieurs amorceurs de polymérisation et d'une solution aqueuse des monomères choisis puis à éliminer tout solvant jusqu'à un taux de siccité de 80 ± 10 % en poids en isolant ainsi le polymère recherché.

Ce polymère convient comme agent absorbant.

## Description

## Polymère hydrophile à base d'acide acrylique et d'acrylate de métal alcalin, son procédé de préparation et son application comme agent absorbant, en particulier d'une solution physiologique.

La présente invention concerne un polymère hydrophile, son procédé de préparation et son application comme agent absorbant.

On connaît des substances polymères susceptibles d'absorber plusieurs fois leur propre poids d'eau en se transformant en gel. Ces polymères hydrophiles sont soit des polymères naturels ou semi-synthétiques tels que des dérivés de la cellulose, de l'amidon, de l'alginate, de polysaccharides, soit des polymères synthétiques à base notamment d'acide maléique ou d'acide (méth)acrylique.

On connaît ainsi une grande variété de polymères hydrophiles mais pour des usages agricoles, horticoles ou sanitaires, on recherche toujours des produits présentant une capacité d'absorption élevée non seulement pour l'eau, mais également pour de l'eau chargée en électrolytes, avec une vitesse d'absorption rapide et un bon pouvoir de rétention à l'état de gel. Par ailleurs, pour l'usage hygiénique, notamment dans la fabrication d'objets destinés à être en contact, à l'état humide, avec l'épiderme humain, on recherche des produits atoxiques, peu onéreux, ne présentant pas de synérèse et ne contenant ni dérivés azotés ni monomères résiduels. On peut dire, aujourd'hui, qu'aucun de ces polymères hydrophiles connus ne répond aux besoins exprimés par le marché.

Or la Demanderesse a découvert de façon surprenante un tel produit.

La présente invention concerne donc un polymère hydrophile, en microperles, insoluble dans l'eau, à base d'acide acrylique et d'acrylate de métal alcalin, présentant une capacité d'absorption de solution physiologique salée élevée, de l'ordre de 50 à 70 g par gramme, caractérisé par le fait qu'il est constitué uniquement, à l'exclusion de tout autre monomère, d'un copolymère acide acrylique - acrylate de potassium, contenant en proportions molaires de 55 à 80 % d'acrylate de potassium.

Parmi les copolymères définis ci-dessus, on peut citer plus particulièrement les copolymères acide acrylique - acrylate de potassium contenant en proportions molaires de 60 à 70 % d'acrylate de potassium et parmi ceux-ci, les copolymères acide acrylique - acrylate de potassium 35-65 en proportions molaires.

Par solution physiologique salée, on désigne une solution isotonique contenant 9 g de chlorure de sodium par litre d'eau distillée.

Par l'expression "à l'exclusion de tout autre monomère", on entend l'absence, même à très faibles doses, de tout produit possédant dans sa structure une fonction chimique susceptible de réagir avec l'acide acrylique, l'acrylate de potassium ou le copolymère acide acrylique - acrylate de potassium.

Selon l'invention, les copolymères définis ci-dessus sont susceptibles d'être préparés par un procédé de polymérisation en suspension eau dans huile réalisé en atmosphère inerte, caractérisé par le fait que l'on introduit lentement, sous agitation, dans la phase huile, parfaitement désoxygénée, maintenue à l'ébullition, et contenant un colloïde protecteur, une solution obtenue extemporanément au fur et à mesure de son introduction à partir, d'une part, d'une solution aqueuse désoxygénée contenant un ou plusieurs amorceurs de polymérisation hydrosolubles présentant une demi-vie à 80°C supérieure à deux heures, et d'autre part, une solution aqueuse contenant à une concentration de 60 ± 5 % en poids, les monomères choisis puis, lorsque la réaction de polymérisation est terminée, que l'on élimine par distillation azéotropique les solvants jusqu'à obtention d'une suspension présentant un taux de siccité d'environ 80 ± 10 % en poids et qu'enfin l'on isole le copolymère cherché par filtration.

La phase huile est constituée par un hydrocarbure tel que le cyclohexane.

Le colloïde protecteur est choisi parmi ceux couramment utilisés dans ce type de polymérisation en suspension (cf. Kirk-Othmer, Encyclopedia of Chemical Technology, 3ème édition, volume 1, page 400). Avantageusement, on choisira un éther de cellulose et, préférentiellement, un éthyléther de cellulose présentant un taux d'éthoxyle de 48 à 49,5 % (cf. Encyclopedia of Polymer Science and Engineering, 2ème édition, volume 3, page 254). Le colloïde protecteur est préalablement dissous ou dispersé dans la phase huile.

La réaction de polymérisation est amorcée par un ou plusieurs générateurs de radicaux libres, hydrosolubles, présentant une demi-vie à 80°C supérieure à deux heures. De tels agents d'amorçage sont notamment certains peroxydes minéraux comme le peroxodisulfate de sodium ou certains azoïques comme l'acide dicyano-4,4' azopentanedioïque-4,4'. L'agent ou les agents d'amorçage utilisés sont dissous dans de l'eau, puis cette solution est soigneusement désoxygénée.

L'acrylate de potassium est obtenu avantageusement en solution aqueuse, par salification directe d'une solution aqueuse d'acide acrylique avec de la potasse.

Cette salification est avantageusement effectuée à une température comprise entre 20°C et 35°C. Les monomères utilisés sont dissous dans de l'eau à une concentration d'environ 60 ± 5 % en poids.

Les solutions aqueuses d'amorçage et de monomères sont mélangées extemporanément, en atmosphère inerte, au fur et à mesure de leur introduction dans la phase huile agitée, parfaitement désoxygénée et maintenue à l'ébullition par un chauffage extérieur si nécessaire.

Ces solutions ne restent donc en contact que quelques secondes avant leur utilisation et elles sont introduites lentement, dans la milieu réactionnel à l'ébullition. La durée d'introduction peut varier selon les unités opératoires, mais généralement elle est comprise entre une et deux heures. En fin d'introduction, il est avantageux de maintenir le milieu réactionnel à l'ébullition sous agitation pour parfaire la polymérisation.

Au cours de la réaction de polymérisation, les copolymères formés se réticulent mutuellement spontanément pour fournir des copolymères réticulés insolubles dans l'eau, à fort pouvoir hydrophile et à très faibles taux de monomères résiduels, lesquels sont toujours inférieurs à 0,01 % en poids.

La réticulation est d'autant plus favorisée que le degré de neutralisation de l'acide acrylique est bas, que la température de polymérisation est plus élevée et que la durée de demi-vie du ou des agents d'amorçage est plus longue. Les copolymères de la présente invention sont donc réticulés thermiquement et de ce fait, deviennent insolubles dans l'eau et acquièrent des propriétés hydrophiles.

Par ailleurs, l'homme du métier sait que les masses moléculaires des copolymères formés sont d'autant plus élevées que la concentration initiale en monomères dans la phase aqueuse est élevée et que la concentration en agent d'amorçage est plus faible. Si bien que l'homme du métier a, à sa disposition, un ensemble de paramètres cinétiques pour moduler à la demande les propriétés du copolymères cherché. Ces propriétés, qui sont celles des réseaux de polymères réticulés, sont accessibles aisément par un ensemble de tests simples qui permettent de modifier, si nécessaire, les conditions opératoires pour atteindre le but recherché.

Ainsi la capacité d'absorption d'eau du polymère est déterminée à 20°C, en agitant durant 30 minutes, 0,4 g de polymère dans 500 g d'eau, puis en pesant le gel polymère obtenu égoutté. Le poids trouvé est ramené à 1 g de polymère. Les copolymères de la présente invention présentent dans ce test une capacité d'absorption de l'ordre de 500 à 800 g par gramme.

La capacité d'absorption de solution physiologique salée du polymère est déterminée à 20°C, en agitant durant 30 minutes, 2 g de polymère dans 500 g de solution physiologique salée puis en pesant le gel polymère obtenu égoutté. Le poids trouvé est ramené comme précédemment à 1 g de polymère. Les copolymères de la présente invention présentent dans ce test une capacité d'absorption de l'ordre de 50 à 70 g par gramme.

La vitesse d'absorption de solution physiologique salée du polymère est déterminée à 20°C, dans un bécher de 100 ml, de diamètre 55 mm et de hauteur 70 mm, en agitant à la vitesse de 600 tours par minute avec un agitateur magnétique équipé d'un barreau aimanté de 25 mm de longueur, 2 g de polymère dans 50 g de solution physiologique salée et en mesurant le temps nécessaire à la disparition du vortex. Dans ce test, les copolymères de la présente invention demandent 30-60 secondes pour la disparition du vortex.

Le taux d'extractibles est déterminé selon la méthode suivante :
- on place 1 g de polymère à tester dans 200 g de solution physiologique salée ;
- on agite cette suspension une heure à 20°C, puis on l'abandonne 15 heures au repos à 20°C ;
- on égoutte le gel polymère obtenu et on recueille le filtrat ;
- on dose sur 100 cm$^3$ du filtrat les fonctions carboxyliques et carboxylates présentes ;
- en exprime le résultat de ce dosage en gramme de polymère dissous pour 100 g de polymère sec.

Dans ce test, les copolymères de la présente invention présentent un taux d'extractibles de 5-15 %. En fin de réaction de polymérisation, on élimine par distillation azéotropique les solvants réactionnels jusqu'à obtention d'une suspension présentant un taux de siccité de 80 ± 10 % puis la suspension est filtrée et le précipité recueilli est séché à 90-95 % de siccité.

On isole ainsi les copolymères selon la présente invention sous forme de perles de quelques dixièmes de millimètre de diamètre, exemptes de fines.

Les copolymères de la présente invention présentent donc d'intéressantes propriétés absorbantes qui justifient leur application comme agent absorbant et l'invention a également pour objet à titre d'agents absorbants les copolymères tels que définis précédemment, notamment pour la fabrication de couches pour bébé.

Les exemples suivants illustrent l'invention sans toutefois la limiter.

Exemple 1

On disperse en atmosphère inerte :
- 227,2 g d'éthyléther de cellulose, contenant de 48 à 49,5 % de groupements éthoxylés et présentant à 25°C, une viscosité de 200 mPa.s en solution à 5 % dans un mélange toluène-éthanol 80-20 en poids,

dans 22723 g (270 moles) de cyclohexane.

Dans cette dispersion, soigneusement désoxygénée, agitée et maintenue à l'ébullition, on introduit en 90 minutes en atmosphère inerte, une solution obtenue par mélange extemporané au cours de l'introduction :
- d'une part, d'une solution désoxygénée de :
. 9,524 g (40 mmoles) de peroxodisulfate de sodium dissous dans 144 g d'eau (8 moles) ;
- d'autre part, d'une solution préparée extemporanément en dissolvant à une température inférieure à 30°C :
. 7206 g (100 moles) d'acide acrylique dans
. 8759 g d'une solution aqueuse de potasse contenant 3647 g (65 moles) d'hydroxyde de potassium et 5112 g d'eau (284 moles).

La suspension obtenue est ensuite maintenue une heure à l'ébullition sous agitation, puis elle est soumise à une distillation azéotropique jusqu'à un taux pondéral de 80 % environ en polymère et, enfin, elle est refroidie à la température ambiante et filtrée. Le précipité est ensuite séché à 90-95 % de siccité sous vide à 60°C.

On obtient ainsi 9675 g de copolymère acide acrylique - acrylate de potassium, 35-65 en proportions molaires, insolubles dans l'eau, et se présentant sous forme de perles de quelques dixièmes de millimètre de diamètre. Ce copolymère présente un taux de monomères résiduels inférieure à 0,005 % en poids, un taux d'extractibles de 12 % en poids, une capacité d'absorption d'eau d'environ 650 g par gramme, une capacité d'absorption de solution physiologique salée d'environ 60 g par gramme et une vitesse d'absorption de solution physiologique salée de 35 ± 5 secondes.

## Exemple 2

On disperse en atmosphère inerte :
- 235,3 g d'éthyléther de cellulose, contenant de 48 à 49,5 % de groupements éthoxylés et présentant à 25°C, une viscosité de 200 mPa.s en solution à 5 % dans un mélange toluène-éthanol 80-20 en poids,

dans 22807 g de cyclohexane.

Dans cette dispersion, soigneusement désoxygénée, agitée et maintenue à l'ébullition, on introduit en 90 minutes en atmosphère inerte, une solution obtenue par mélange extemporané au cours de l'introduction :
- d'une part, d'une solution aqueuse désoxygénée de :
. 10,24 g (43 mmoles) de peroxodisulfate de sodium dissous dans 144 g d'eau,
- d'autre part, d'une solution préparée extemporanément en dissolvant à une température inférieure à 30°C :
. 7206 g (100 moles) d'acide acrylique dans
. 8680 g d'une solution aqueuse de potasse contenant :
- 3928 g (70 moles) d'hydroxyde de potassium et
- 4752 g (264 moles) d'eau.

La suspension obtenue est ensuite maintenue une heure à l'ébullition sous agitation, puis elle est soumise à une distillation azéotropique jusqu'à un taux pondéral de 80 % environ en polymère, et enfin elle est refroidie à la température ambiante et elle est filtrée. Le précipité recueilli est ensuite séché à 90-95 % de siccité sous vide à 60°C.

On obtient ainsi 9865 g de copolymère acide acrylique - acrylate de potassium, 30-70 en proportions molaires, insoluble dans l'eau et se présentant sous forme de perles de quelques dixièmes de millimètre de diamètre. Ce copolymère présente un taux de monomères résiduels inférieur à 0,005 % en poids, une capacité d'absorption d'eau d'environ 750 g par gramme, une capacité d'absorption de solution physiologique salée d'environ 68 g par gramme et une vitesse d'absorption de solution physiologique salée de 55 ± 5 secondes.

Il va de soi que la présente invention n'a été décrite qu'à titre purement explicatif et nullement limitatif et que toute modification, notamment au niveau des équivalences techniques, pourra y être apportée sans sortir de son cadre.

## Revendications

1. Polymère hydrophile, insoluble dans l'eau, caractérisé par le fait qu'il est à base d'acide acrylique et d'acrylate de métal alcalin, qu'il présente une capacité d'absorption de solution physiologique salée de l'ordre de 50 à 70 g par gramme, et qu'il est constitué uniquement de 20 à 45 % en proportions molaires d'acide acrylique et de 80 à 55 % en proportions molaires d'acrylate de potassium à l'exclusion de tout autre monomère.

2. Polymère hydrophile selon la revendication 1, caractérisé par le fait que c'est un copolymère acide acrylique - acrylate de potassium contenant en proportions molaires de 60 à 70 % d'acrylate de potassium.

3. Polymère selon la revendication 1 ou 2, caractérisé par le fait qu'il se présente sous forme de perles.

4. Procédé de préparation d'un polymère hydrophile selon l'une quelconque des revendications 1 à 3, caractérisé par le fait que c'est un procédé de polymérisation en suspension eau dans huile, réalisé en atmosphère inerte, du type suivant lequel on introduit lentement sous agitation dans la phase huile, parfaitement désoxygénée, maintenue à l'ébullition et contenant un colloïde protecteur, une solution obtenue extemporanément, au fur et à mesure de son introduction, à partir d'une part, d'une solution aqueuse contenant un ou plusieurs amorceurs de polymérisation hydrosolubles présentant une durée de demi-vie à 80°C supérieure à deux heures et, d'autre part, une solution aqueuse contenant à une concentration de 60 ± 5 % en poids les monomères choisis puis, lorsque la réaction de polymérisation est terminée, on élimine les solvants par distillation azéotropique jusqu'à un taux de siccité de 80 ± 10 % en poids et enfin on isole le polymère cherché par filtration.

5. Procédé selon la revendication 4, caractérisé par le fait que la colloïde protecteur est un éthyléther de cellulose.

6. Application comme agent absorbant d'un polymère hydrophile tel que défini à l'une quelconque des revendications 1 à 3.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, vol. 82, no. 10, 19 mai 1975, page 20, résumé no. 125874n, Columbus, Ohio, US; & JP-A-74 105 889 (NIHON PURE CHEMICAL CO., LTD) 07-10-1974 --- | | C 08 F  20/06 A 61 L  15/00 |
| A | GB-A-2 093 351  (KAO SOAT CO.) ----- | | |

**DOMAINES TECHNIQUES RECHERCHES (Int. Cl.4)**

C 08 F

Le présent rapport a été établi pour toutes les revendications

| Lieu de la recherche | Date d'achèvement de la recherche | Examinateur |
|---|---|---|
| LA HAYE | 31-08-1988 | CAUWENBERG C.L.M. |

EPO FORM 1503 03.82 (P0402)